# EUROPEAN PATENT APPLICATION

(11) **EP 4 621 378 A2**
(43) Date of publication of application: **24.09.2025**
(21) Application number: 25165179.0
(22) Date of filing: 20.03.2025
(51) Int. Cl.: G01N 1/28, A61B 90/20

(54) **TISSUE SPECIMEN MARKING DEVICES AND METHOD OF MARKING TISSUE SPECIMENS**

(30) Priority: 22.03.2024 US 202463568940 P
(71) Applicant: Perimeter Medical Imaging AI, Inc., Toronto, Ontario M5V 3B1 (CA)
(72) Inventor: FAN, Chao, Toronto, M5V 3B1 (CA); SANATI-BURNS, Maggie, Toronto, M5V 3B1 (CA); SHARMA, Anshul, Toronto, M5V 3B1 (CA); GAZDZINSKI, Carl, Toronto, M5V 3B1 (CA); BERKELEY, Andrew, Toronto, M5V 3B1 (CA)
(74) Representative: Potter Clarkson

(57) **Abstract**

Tissue marking devices and methods of tissue marking. Tissue marking devices may include: an elongate body member; an applicator head coupled to the elongate body member having a parabolic profile; and an elongate nib coupled to a distal edge of the applicator head, the elongate nib including a nib profile corresponding to the parabolic profile of the applicator head and adapted to dispense ink.

## Description

### FIELD

Embodiments of the present disclosure generally relate to the field of tissue specimen analysis and, in particular, to tissue specimen marking devices.

### BACKGROUND

Surgical oncology is directed to surgical management of cancerous tumors. In some situations, excised tissue specimens from cancer patients may be examined based on operations for microscopic pathological examination or other types of examination. In some examples, excised tissue specimens may have multiple surfaces, akin to a volumetric prism.

### SUMMARY

Tissue specimens may be fragile and may have variable solidity. Examples of tissue specimen may include adipose tissue, among other examples of tissue from various organs. In some scenarios, tissue specimens may have a volumetric shape that may change when rotated from one spatial orientation to a subsequent spatial orientation. It may be desirable to provide marking devices adapted to generate markings proximal to incisions of excised tissue specimens for identifying tissue specimen margins. Such ink markings may be adapted to record spatial orientation data associated with tissue specimen margins.

Features of embodiments of marking devices and methods of tissue marking and of tissue specimen analysis will be described in the present disclosure.

In one aspect, the present disclosure describes a tissue marking device. The tissue marking device may include: an elongate body member; an applicator head coupled to the elongate body member having a parabolic profile; and an elongate nib coupled to a distal edge of the applicator head, the elongate nib including a nib profile corresponding to the parabolic profile of the applicator head and adapted to dispense ink.

In another aspect, the present disclosure describes a method of tissue marking. The method of tissue marking may include: determining an incision edge associated with a tissue specimen margin; applying an ink segment along the incision edge with a tissue marking device, the tissue marking device including an applicator head having a parabolic profile and an elongate nib coupled to a distal edge of the applicator head, wherein the elongate nib including a nib profile corresponding to the parabolic profile of the applicator head and adapted to dispense ink; and orienting the tissue specimen to advance to a subsequent spatial orientation for marking an adjacent tissue margin.

In another aspect, the present disclosure describes a method of tissue specimen analysis. The method of tissue specimen analysis may include: immobilizing a first tissue specimen margin to be in contact with an imaging surface of an imaging receptacle, the tissue specimen margin having at least one ink marking thereon; determining a spatial orientation of the tissue specimen based on the identified ink marking, the identified ink marking including at least one of an ink segment corresponding to an incision edge of the tissue specimen or an indicia mark on the first tissue specimen margin; generate a signal representing spatial orientation data for display to correlate the spatial orientation data with a surgical location at a patient for the first tissue specimen margin.

In this respect, before explaining at least one embodiment in detail, it is to be understood that the embodiments are not limited in application to the details of construction and to the arrangements of the components set forth in the following description or illustrated in the drawings. Also, it is to be understood that the phraseology and terminology employed herein are for the purpose of description and should not be regarded as limiting.

Many further features and combinations thereof concerning embodiments described herein will appear to those skilled in the art following a reading of the present disclosure.

### DESCRIPTION OF THE FIGURES

In the figures, embodiments are illustrated by way of example. It is to be expressly understood that the description and figures are only for the purpose of illustration and as an aid to understanding.

Embodiments will now be described, by way of example only, with reference to the attached figures, wherein in the figures:
FIG. 1 illustrates a perspective view of an excised tissue specimen, in accordance with an embodiment of the present disclosure;
FIG. 2 illustrates a perspective view of an excised tissue specimen, in accordance with an embodiment of the present disclosure;
FIG. 3 illustrates a coded indicia markings for tissue specimen marking operations, in accordance with embodiments of the present disclosure;
FIG. 4 illustrates a perspective view of a marking device, in accordance with embodiments of the present disclosure;
FIG. 5 illustrates an enlarged rear view of an applicator head, in accordance with an embodiment of the present disclosure;
FIG. 6 illustrates an enlarged view of an indicia head, in accordance with an embodiment of the present disclosure;
FIG. 7 illustrates a photo showing a marking device for generating ink segments on a tissue specimen, in accordance with embodiments of the present disclosure;
FIG. 8 illustrates a tissue analysis system, in accordance with an embodiment of the present disclosure;
FIG. 9 illustrates an image representing a tissue surface generated by the tissue analysis system of FIG. 8, in accordance with an embodiment of the present disclosure;
FIG. 10 illustrates a flowchart of a method of tissue marking, in accordance with an embodiment of the present disclosure; and
FIG. 11 illustrates a flowchart of a method of tissue specimen analysis, in accordance with an embodiment of the present disclosure.

### DETAILED DESCRIPTION

Embodiments of the present disclosure are directed to devices and methods of marking excised tissue specimens and systems and methods of image specimen analysis.

For cancer patients, a first line of treatment is a surgical removal of an identified tumor or tissue. In the field of surgical oncology, surgeons or medical staff may remove tissue specimens from patients during a surgical procedure. It may be desirable to determine during a surgical procedure whether an identified cancerous lesion has been entirely removed while a surgical patient is still within an operating room environment.

Once a tumor is removed, operations for validating that removed tissue specimens do not have cancerous cells at an excised tissue margin may be desirable for patient prognosis. In some scenarios, identification of cancerous cells at an excised tissue margin may be identified as a positive margin.

In some scenarios, positive margins may increase locoregional recurrence rates in patients with breast, colorectal, oral cavity, bladder, or uterine cancer, among other types of conditions. In some scenarios, positive surgical margins may decrease disease-specific survival rates in patients with breast or bladder cancer, and may decrease overall survival rate in colorectal, oral cavity, or lung cancer patients.

Tissue specimens may be amorphous in nature. In some examples, excised tissue specimens may have multiple surfaces, akin to a volumetric prism. In some scenarios, excised tissues can have up to 6 sides. For instance, an excised tissue specimen may vary in size from having 1 centimeter to 10-centimeter side lengths and the excised tissue specimen may resemble a cubic shaped volume with six general sides, including an anterior side, posterior side, superior side, inferior side, medial side, or lateral side.

Once excised tissue specimens are removed from a patient, tissue edges where a surgeon may have made incisions for removing the tissue specimen may not maintain clearly defined edges. It may be desirable to provide markings at incision edges or surfaces such that a spatial orientation of the tissue sample may be recorded. For example, in the event that downstream analysis shows that cancerous cells may be at or near the incision edge or surface of a tissue specimen, surgeons may wish to conduct a subsequent surgical procedure to remove additional patient tissue corresponding to the incision edge or surface correlated with the identified cancerous cells.

In some scenarios, surgeons or medical staff may create markings on the excised tissue specimen to provide guidepost or location markers relative to the location where the excised tissue was removed. For example, surgeons may utilize specifically placed sutures to provide a location marker, such as for identifying a superior or lateral side of the tissue specimen. A short suture may be affixed to a location to represent a superior side and a long suture may be affixed to a location to represent a lateral side. In some scenarios, a third suture may be affixed to another location in combination with the above-described sutures.

In scenarios where sutures are positioned for providing landmarks or guideposts, excised tissues specimens may be placed in a solution that includes 10% formalin for transporting to a facility for pathology analysis. Use of sutures in the present example may be suitable to provide 2 landmarks for two tissue specimen margins. However, providing landmarks at two locations for a three-dimensional volume may be insufficient for providing medical staff with sufficient spatial orientation data for identifying specimen margins relative to a user's anatomy after a surgical procedure is concluded.

As an example, surgeons may need two sutures and a marking to identify laterality of excised breast tissue, such as left or right. If lateral and superior is identified, a surgeon or pathologist may require information on whether the excised breast tissue is from a left side or a right side for determining anterior and posterior margin surfaces. Accordingly, utilizing 2 landmarks may be insufficient for defining excised tissue margins. Further, excised tissue marked with sutures with a goal of marking boundaries may be interpreted differently by different pathologists.

In some scenarios, tissue specimen marking may include operations for applying distinctive colored ink on substantial portions of a plurality of tissue specimen surfaces. For example, different colored ink may be used on adjacent tissue specimen surfaces to distinguish an anterior side from a posterior side. Such inking methods may be time consuming or cumbersome and may necessitate time for the applied ink to adhere or dry on respective surfaces of the tissue specimen. While tissue specimen surfaces may be identifiable, the applied ink substantially coating the tissue surface may hinder downstream imaging operations configured for visually determining whether cancerous cells may be proximal the tissue specimen surface or margins. For example, imaging devices may be unable to comprehensively generate image data of excised tissue margins if the tissue margin surface is substantially coated in ink.

In some scenarios, tissue specimen marking may be based on affixing clips to the tissue specimen at a desired location at the surface of the tissue specimen. In some environments, clips may inadvertently be unaffixed when the tissue specimen is physically repositioned, manipulated or transported. In some scenarios, affixing clips to the tissue specimen may cause physical alterations to the tissue specimen. For example, affixed clips may inadvertently pinch tissue and alter physical structure of the tissue surface. In some scenarios, clips configured as landmarks may not provide delineation among a plurality of surfaces of the tissue specimen. Clips may simply be single landmark points on the tissue specimen surface.

In some embodiments, a system may include image capture devices for generating images of excised tissue specimens, and the system may include operations for determining based on generated images whether cancerous cells may be present at the tissue specimen margins. In some scenarios, a surgeon or medical team staff may manipulate the tissue specimen by flipping the tissue a plurality of times to generate images of the plurality of surfaces (e.g., akin to imaging 6 sides of dice).

Because tissue specimens may be amorphous in nature, when the tissue specimens are manipulated for generating images of the surfaces, two or more images may include image data of overlapping regions because tissue specimens may flex when placed atop a surface for imaging.

It may be desirable to provide tissue marking devices and methods for intraoperatively marking excise tissue specimens for preserving spatial orientation data for allowing downstream operations for correlating a tissue specimen surface with a location on a patient's organ.

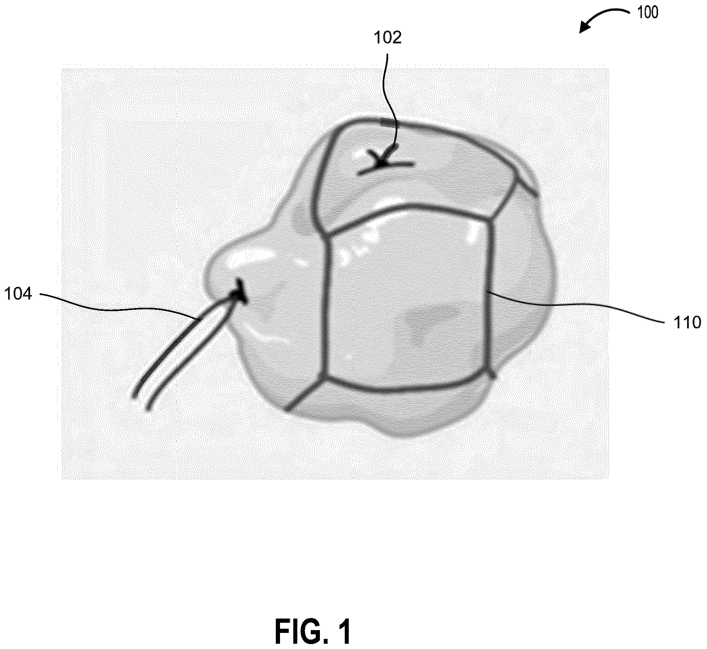

Reference is made to FIG. 1, which illustrates a perspective view of an excised tissue specimen 100, in accordance with an embodiment of the present disclosure. The tissue specimen 100 may be a volume of tissue. The tissue specimen 100 may have been removed from a patient.

In some scenarios, the excised tissue specimen 100 may be a volume having one or more sides. In some scenarios, the tissue specimen may be amorphous and upon being removed from a patient may not have clearly defined edges or sides. It may be desirable to provide tissue markings to assist with identifying an edge or border of tissue removed from a patient.

In a scenario where the margin may be identified as potentially having cancer cells (e.g., a "positive margin"), a surgeon or medical team may wish to identify the location at a patient that corresponds to the identified positive margin. The knowledge may be desirable so that the surgeon or medical team may subsequently revisit the location of the user's organ corresponding to the positive margin and remove further tissue with a goal to removing substantially all cancerous cells.

The excised tissue specimen 100 shows a short suture 102 positioned at a first surface of the tissue specimen 100 and a long suture 104 at a second surface of the tissue specimen 100. In some scenarios, surgeons may thread and place sutures at landmark locations of the tissue specimen 100. As described above, providing landmarks at two locations of a three-dimensional volume may be insufficient for providing medical staff with spatial orientation data for identifying specimen margins relative to a user's anatomy after excised tissue specimens are removed from the patient. As described above, boundaries between margins may not be well defined based on landmarks at two locations of a three-dimensional volume, leading to pathologists conducting their best interpretation for spatial orientation data.

In some embodiments, the excised tissue specimen 100 may be marked with ink segments 110. A surgeon or medical staff member may generate the ink segments 110 at locations substantially corresponding to one or more interfaces where a surgeon make incisions for removing the excise tissue specimen 100.

In some embodiments, a plurality of ink segments 110 may circumscribe a portion of surface area of the excised tissue specimen 100. In examples where the excised tissue specimen 100 may resemble a cubic volume or solid having 6 surfaces, the plurality of ink segments 110 may delineate respective surfaces of the tissue specimen 100 from the other five surfaces.

In some scenarios, a surgeon may utilize the short suture 102 or the long suture 104 as gripping points for manipulating the tissue specimen 100 into a desired orientation for downstream imaging operations.

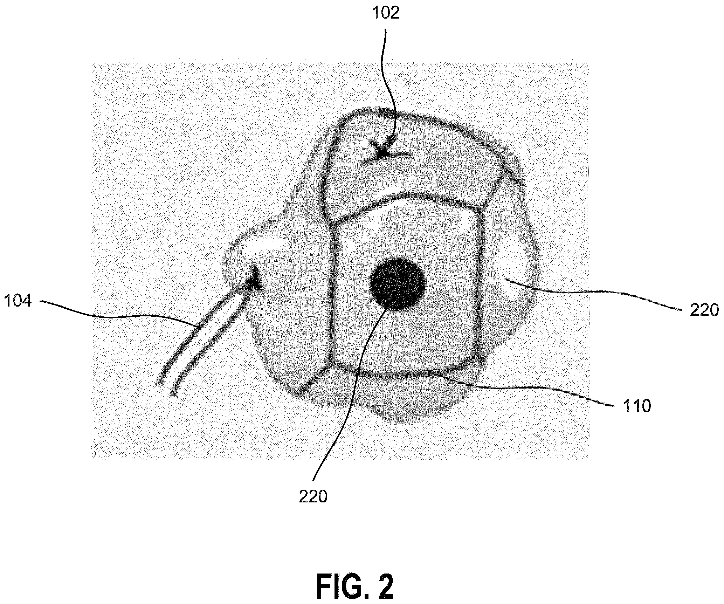

In some scenarios, it may be desirable to provide indicia markings for distinguishing a tissue specimen surface from another of the plurality of tissue specimen surfaces. Reference is made to FIG. 2, which illustrates the excised tissue specimen 100 shown in FIG. 1.

In addition to generating ink segments 110 at locations substantially corresponding to one or more edges where tissue incisions may have been made, or at any other desired surface location of the tissue specimen 100, in some scenarios, a surgeon may generate identifying surface markers 220 for distinguishing respective surfaces of the tissue specimen 100 from other surfaces of the tissue specimen 100.

For example, it may be desirable to for a surgeon or the medical team to be able to expediently distinguish an anterior side from an inferior side of the tissue specimen 100 once the tissue specimen 100 has been removed from a patient. Accordingly, the generated surface markers 220 may be distinguishing indicia, including distinguishing-colored markings or other distinguishing coded indicia for correlating a surface area of the tissue specimen 100 with an anatomical location of the user from which the tissue specimen 100 was removed.

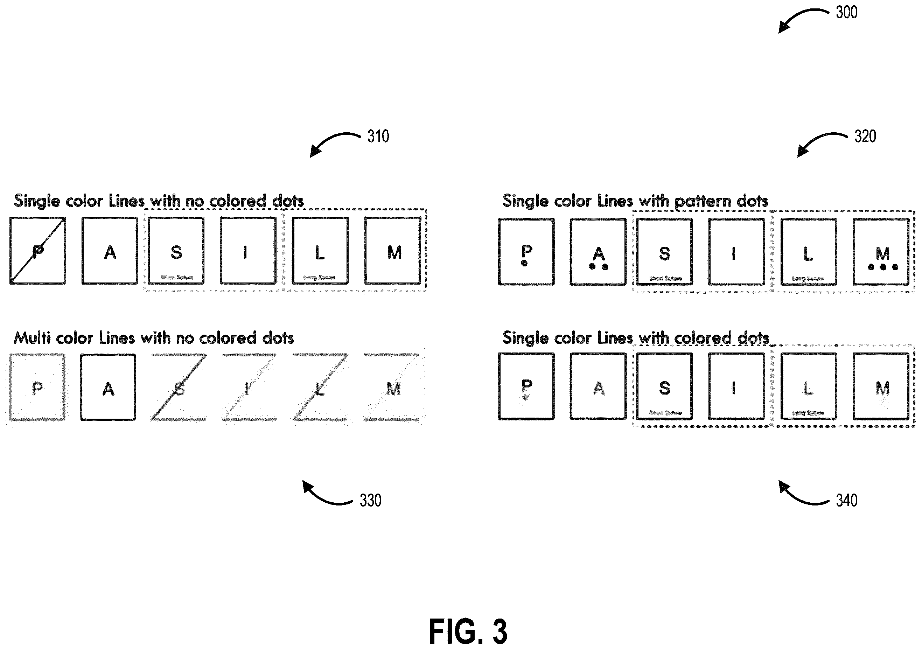

Reference is made to FIG. 3, which illustrates coded indicia markings 300 for tissue specimen marking operations, in accordance with embodiments of the present disclosure. For example, a first set of coded indicia markings 310 may include a single-color set of marking segment lines.

As another example, a second set of coded indicia markings 320 may include a single-color set of marking line segments in combination with patterned dots.

As another example, a third set of coded indicia markings 330 may include a set of multiple-colored lines for marking on tissue specimen surfaces for distinguishing respective surfaces from other tissue specimen surfaces.

As another example, a fourth set of coded indicia markings 340 may include a single-color set of marking segment lines in combination with colored dots.

Surgeons may be tasked with removing tissue specimens, such as breast tissue, colon tissue, lung tissue, or tissues from other types of organs. Tissue specimens may be amorphous in nature. When tissue is removed from a patient, the tissue specimen may lack a regular geometric structure or shape and may deform based on external forces such as gravitational forces or external impetus (e.g., a surgeon manipulating the tissue specimen).

Because of the amorphous nature of tissue specimens, it may be desirable to provide devices for intraoperatively marking tissue specimens. In some scenarios, it may be desirable to provide devices and methods for efficiently generating marking segments on tissue specimens having an amorphous structure. Such marking segments may provide spatial orientation data for corresponding the geometric features of the tissue specimen with an organ location from which the tissue specimen was removed from the user.

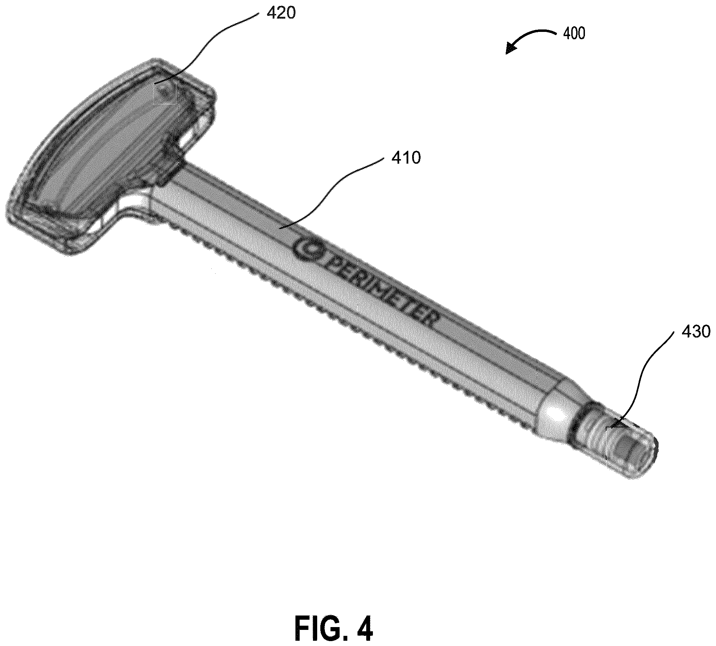

Reference is made to FIG. 4, which illustrates a perspective view of a marking device 400, in accordance with embodiments of the present disclosure. The marking device 400 may be a hand-held device intraoperatively used for applying one or a plurality of ink segments on tissue specimens. In some scenarios, the marking device 400 may be manipulated by a surgeon concurrently with cutting devices such that ink segments are applied on the tissue specimen whilst incisions are made for removing the tissue specimen.

The marking device 400 may include a body member 410. In some embodiments, the body member 410 may be an elongate handle, such as a barrel member, for a surgeon to manipulate in a way similar to methods of utilizing a pen. In some embodiments, the body member 410 may include a plurality of grooves configured to increase friction between the body member 410 and a hand of a user, thereby providing an efficient gripping surface.

In some embodiments, the body member 410 may include chamfered edges for providing ergonomic placement in a surgeon's hand. Other features for providing an efficient or ergonomic gripping surface may be used.

The marking device 400 may include an applicator head 420 at a first end of the body member 410. In some embodiments, the applicator head 420 may be coupled or affixed to the body member 410 and may be adapted for generating ink segments on tissue specimens. A distal edge of the applicator head 420 may be adapted to allow a surgeon to successively generate a plurality of ink segments for marking a surface of a tissue specimen.

In some embodiments, the distal edge of the applicator head 420 may have a parabolic profile such that a surgeon may generate a plurality of ink segments in succession substantially corresponding to the path of incisions made by the surgeon for removing the tissue specimen.

In some embodiments, the marking device 400 may include an indicia head 430. In some embodiments, the indicia head 430 may include a rounded point surface adapted for applying circular dots or similar markings at the tissue specimen. In some embodiments, the indicia head 430 may be adapted to provide a surgeon with a writing instrument for intraoperatively applying indicia to the tissue specimen.

In some embodiments, the marking device 400 may be operated in combination with an ink source, such as an ink receptacle. In some examples, the ink receptacle may be an ink pad or an ink well. To apply ink segments to tissue specimen, the surgeon may manipulate the applicator head 420 or the indicia head 430 to retrieve in from an ink receptacle before applying ink segments or ink indicia to the tissue specimen. Such examples may be desirable in scenarios where point-of-use inking operations may be suitable.

In some embodiments, the applicator head 420 or the indicia head 430 may be adapted to receive a protective cover for insulating the applicator head 420 or the indicia head 430 from the environment when the marking device 400 is not being used.

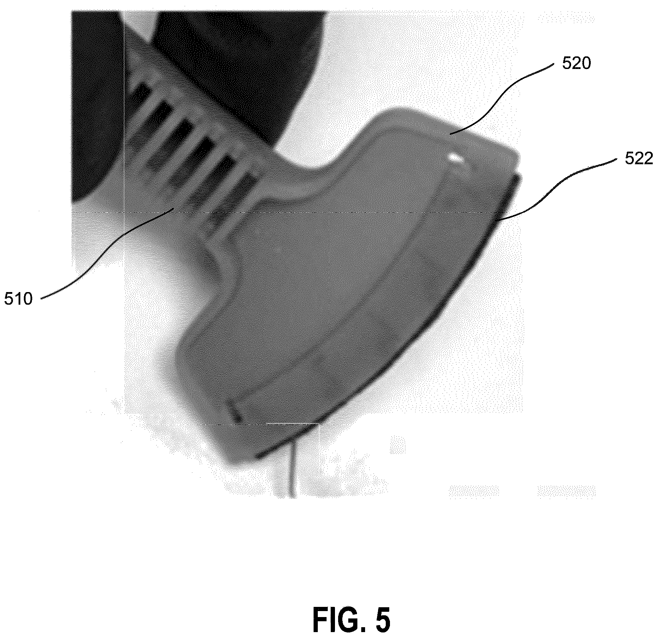

Reference is made to FIG. 5, which illustrates an enlarged rear view of an applicator head 520, in accordance with an embodiment of the present disclosure. The applicator head 520 may be coupled to or affixed to an elongate handle 510.

In some embodiments, the applicator head 520 may have a parabolic profile. The parabolic profile may be dimensioned such that a surgeon user may roll the applicator head 520 along the parabolic profile whilst generating ink segments on the tissue specimen. In some scenarios, the parabolic profile may allow a surgeon user to efficiently slide the applicator head because of the curved shape of the parabolic profile. The parabolic profile at the applicator head 520 may allow the surgeon user to generate successive ink segments that in combination may provide an overall ink segment that is longer than the length along the parabolic profile. The above-described embodiment of the applicator head 520 may be adaptable for generating successive, continuous ink segments for tissue specimens of varying dimensions.

In some embodiments, the applicator head 520 may include a line nib 522 coupled to a distal edge of the applicator head 520. The distal edge of the applicator head 520 may be configured for interfacing with tissue specimens for generating ink segments.

In some embodiments, the line nib 522 may be felt nib 522 adapted for transferring ink to tissue specimens. In some embodiments, the line nib 522 may be constructed of other types of material for transferring ink to tissue specimens.

The line nib 522 may have a parabolic profile substantially similar to a parabolic profile of the applicator head 520. The line nib 522 may have a profile that substantially corresponds to the profile of the applicator head 520.

In some embodiments, the line nib 522 may be pre-inked felt nibs for dispensing ink. The pre-inked felt nibs may include a quantity of ink sufficient for generating ink segments for several tissue specimens. For example, the pre-inked felt nibs may include ink sufficient for marking up to 20 ink segments.

In some embodiments, the line nib 522 may include ink-filled burstable capsules embedded in the line nib 522. Ink-filed burstable capsules may be used for isolating ink from the line nib 522 until substantially such time that a surgeon wishes to generate ink segments on tissue specimens. Line nibs 522 having ink-filled burstable capsules may increase the shelf-life of the marking device as compared to pre-inked felt nibs. If not within sealed packaging, marking devices having line nibs with ink-filled burstable capsules may dry out over time.

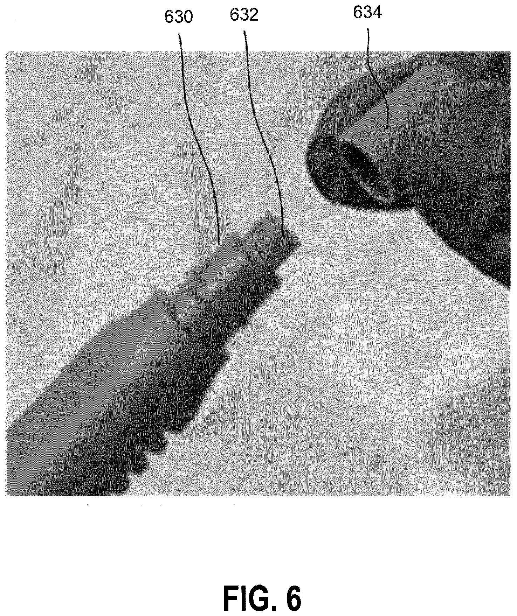

Reference is made to FIG. 6, which illustrates an enlarged view of an indicia head 630, in accordance with an embodiment of the present disclosure. The indicia head 630 may include a point surface for applying circular-shaped dots or other indicia markings to a tissue specimen. In some embodiments, the indicia head 630 may provide a surgeon with a writing instrument for intraoperatively applying indicia to the tissue specimen.

In some embodiments, the indicia head 630 may include a point nib 632 coupled at the indicia head 630. In some embodiments, the point nib 632 may be a felt nib or a nib of other material type adapted to retain ink for generating indicia at tissue specimen.

In some embodiments, the point nib 632 may be pre-inked felt nibs for dispensing ink. The pre-inked felt nibs may include a quantity of ink sufficient for generating ink segments for several tissue specimens. For example, the pre-inked felt nibs may include ink sufficient for marking up to 20 ink segments, or a quantity of ink for more or less ink segment markings.

In some embodiments, the point nib 632 may include ink-filled burstable capsules embedded in the point nib 632. The ink-filed burstable capsules may be used for isolating ink from the point nib 632 until substantially such time that a surgeon wishes to generate ink indicia on tissue segments.

In some embodiments, the point nib 632 may have a cylindrical shape adapted to efficiently generate an ink dot based on the circular-faced nib. In the present example, the cylindrical shape may be amenable to generating a point or 'dot' to mark tissue specimens with a colored dot.

In some embodiments, the indicia head 630 may be adapted to receive a protective cover 634 for insulating the indicia head 630 from the environment when the marking device is not being used.

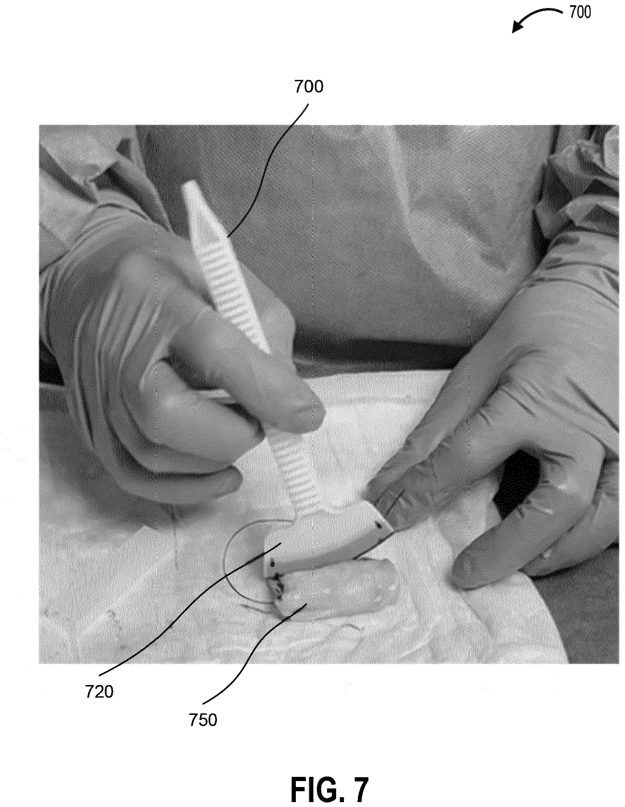

Reference is made to FIG. 7, which illustrates a photo showing marking device 700 for generating ink segments on a tissue specimen 750, in accordance with embodiments of the present disclosure.

In FIG. 7, the marking device 700 may include an applicator head 720 having a parabolic profile. A surgeon manipulating the marking device 700 may roll, in a direction corresponding to a direction of a curve of the parabolic profile, the applicator head 720 along the tissue specimen 750 for generating the ink segment. The surgeon may repeatedly generate ink segments for visually delineating tissue surfaces or margins of the tissue specimen 750. Upon generating ink segments to delineate tissue surfaces that may represent a plurality of tissues margins, the tissue specimen 750 may include a plurality of ink segments similar to the plurality of ink segments illustrated in FIG. 1.

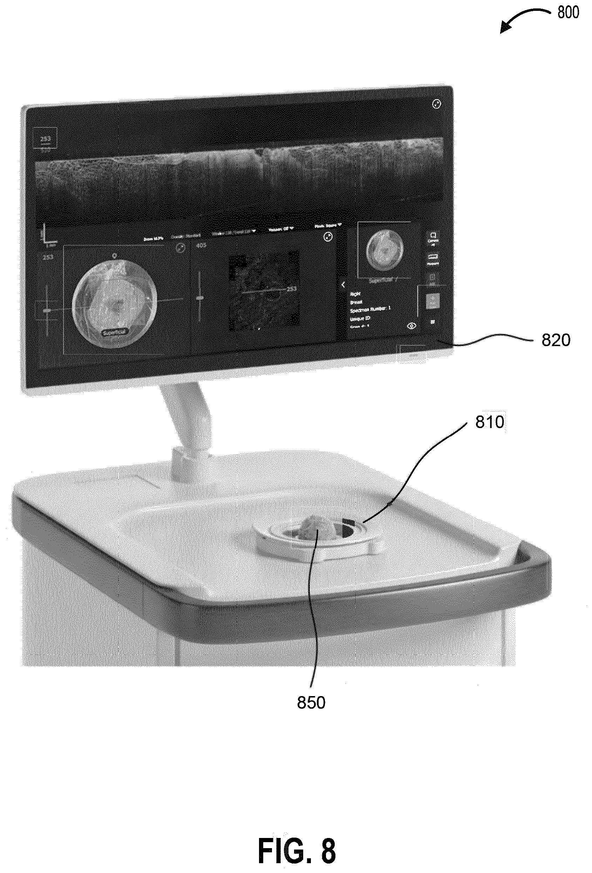

Reference is made to FIG. 8, which illustrates a tissue analysis system 800, in accordance with an embodiment of the present disclosure. The tissue analysis system 800 may include an imaging receptacle 810 and a visual display 820 for displaying imaging data or analytical data associated with a tissue specimen 850 placed within the imaging receptacle 810.

In some embodiments, the tissue analysis system 800 may be positioned within an operating room. The tissue analysis system 800 may be configured to receive tissue specimens 850 during an operation procedure, and the tissue analysis system 800 may conduct operations for imaging the tissue specimen 850 and conducting operations for the respective tissue surfaces or margins for determining whether there may be suspected cancerous cells at or near the surface of the tissue specimen 850. Operations for determining whether there may be suspected cancerous cells at or near the surface of the tissue specimen 850 may be based on image analysis of the tissue specimen 850.

In scenarios where cancerous cells may be inferred to be at or near the surface of the tissue specimen 850, the tissue analysis system 800 may, based on ink indicia previously applied to the tissue specimen 850, identify the spatial orientation of the tissue specimen 850 and provide data to assist the surgical team with identifying an anatomical location at the patient where further tissue may be excised. For example, the ink indicia previously applied to the tissue specimen 850 may be adapted to suggest that the currently imaged tissue surface or margin is of one of the anterior side, posterior side, superior side, inferior side, medial side, or lateral side of the tissue specimen 850. Having an inference of which tissue surface or margin is being imaged may assist the surgical team with identifying a location at the patient's organ for further retrieval of excised tissue.

The tissue analysis system 800 may include the imaging receptacle 810 adapted to receive a tissue specimen 850. Prior to being placed in the imaging receptacle 810, the tissue specimen 850 may have been marked with a plurality of ink segments for visually delineating a plurality of tissue surfaces or margins for analysis. For example, the tissue specimen 850 may have a plurality of ink segments applied thereon akin to operations described with reference to FIG. 7.

During an operating procedure for removing tissue specimens from a patient, a surgeon or the medical team may need to expediently and accurately identify the respective tissue surfaces or margins previously marked and rotate the tissue specimen 850 within the imaging receptacle 810.

In some scenarios, tissue specimens 850 may be fragile and can have variable solidity. The tissue specimen 850 may have a volumetric shape that may change when rotated from one orientation to a subsequent orientation. When tissue specimens 850 may be placed in the imaging receptacle 810, the tissue specimen 850 may collapse onto the imaging surface. In some scenarios, the structural collapse of the tissue specimen 850 may be referred to as a 'pancaking effect'. As the tissue specimen 850 is manipulated, the shape of the tissue specimen 850 may change thereby making it challenging to identify tissue surfaces or margins, even with the aid of sutures or other physical landmarks.

When the tissue specimen 850 is placed within the imaging receptacle 810, the tissue specimen 850 may be physically immobilized. In some embodiments, a vacuum suction device may be adapted to physically immobilize the tissue specimen 850 thereby amplifying the pancaking effect on the tissue specimen 850. Immobilizing the tissue specimen 850 ensures the tissue specimen 850 is in secure contact with the imaging surface glass. As the tissue specimen 850 may be immobilized within the imaging receptacle 810, the plurality of ink segments may be visible within images and may assist with providing delineated markers separating tissue surfaces or margins of the tissue specimen 850.

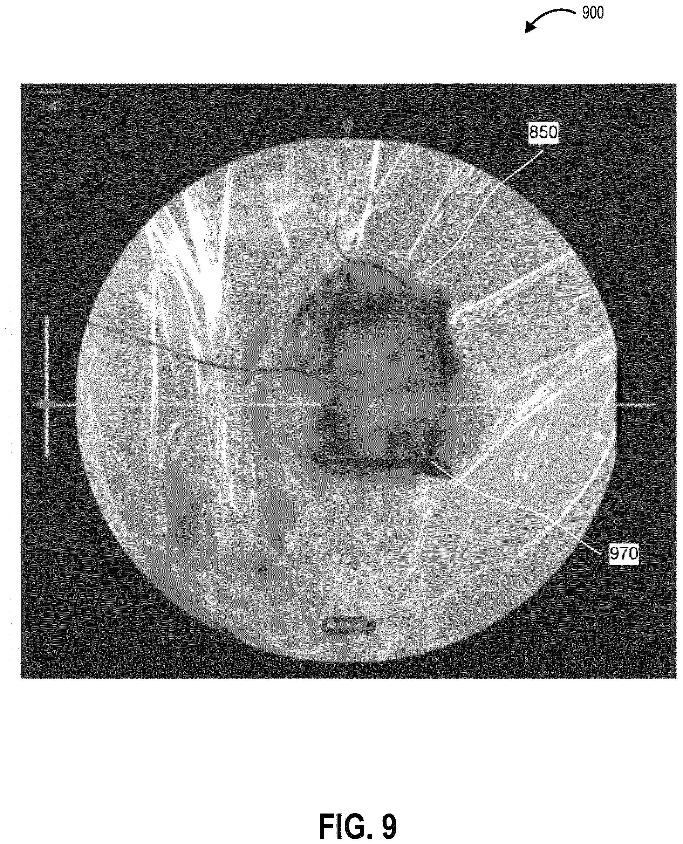

Reference is made to FIG. 9, which illustrates an image 900 representing a tissue surface of the tissue specimen 850 described in FIG. 8. The image 900 may be generated by the tissue analysis system 800 (FIG. 8). The tissue specimen 850 may be within the imaging receptacle. Illustrated tissue specimen 850 may have been immobilized and the tissue specimen 850 may be subject to the pancaking effect such that the tissue surface is in contact with an imaging surface.

In FIG. 9, the image 900 shows a plurality of ink segments 970 circumscribing a tissue surface or margin. In the example illustrated in FIG. 9, the tissue surface is associated with an anterior side of the tissue specimen 850. In some embodiments described herein, methods of generating ink segments 970 or ink indicia associated with respective tissue margins or surfaces may be for providing coded indicia. As such, tissue analysis systems 800 may infer spatial orientation data associated with the tissue margin / surface of the tissue specimen 850.

In scenarios where a surgical team decides to revisit the surgical site for obtaining further tissues from the patient, the surgical team may infer or identify a location at the patient's organ that corresponds to the tissue margin illustrated in the tissue image 900.

In some embodiments, the tissue analysis system 800 (FIG. 8) may include one or more detection models configured to predict whether cancerous cells are at or near a tissue specimen margin based on image analysis. Respective tissue specimen margins may be visually distinguished based on ink segments created based on embodiments of tissue marking devices described in the present disclosure.

In some embodiments, the tissue analysis system 800 may be trained to determine the spatial orientation of the tissue specimen margin based on one or more ink segments or indicia generated at the tissue specimen margin. Some embodiments of the ink segments or ink indicia are depicted and described with reference to FIG. 3.

As described herein, embodiments of a tissue marking device may be configured for generating one or more ink segments or indicia markings at tissue specimens. The ink segments or indicia markings may be for assisting with visually distinguishing respective tissue specimen margins from adjacent tissue specimen margins.

In some embodiments, a tissue marking device may include an elongate body member and an applicator head coupled to the elongate body member. The applicator head may have a parabolic profile. In some scenarios, the applicator head profile may be adapted to assist a surgeon or a medical team member with efficiently generating successive ink segments. For example, an applicator head having a parabolic profile may be adapted to be rocked back and forth while marking along an incision edge of the tissue margin.

In some embodiments, the tissue marking device may include an elongate nib coupled to a distal edge of the applicator head. The elongate nib may include a nib profile corresponding to the parabolic profile of the applicator head and adapted to dispense ink.

In some embodiments, the elongate nib may include an ink-filled burstable capsule. In some embodiments, the applicator head may include a chamfered edge.

In some embodiments, the elongate body member may include a plurality of grooves extending a length of the elongate body member. In some embodiments, the tissue marking device may include a protective cover having a profile substantially similar to the applicator head and adapted to cover the applicator head.

In some embodiments, the tissue marking device may include an indicia head coupled to the elongate body member at a distal end to the application head and a point nib coupled to the indicia head and adapted to dispense ink. In some embodiments, the point nib includes a cylindrical profile. In some embodiments, the point nib includes colored ink absorbed therein.

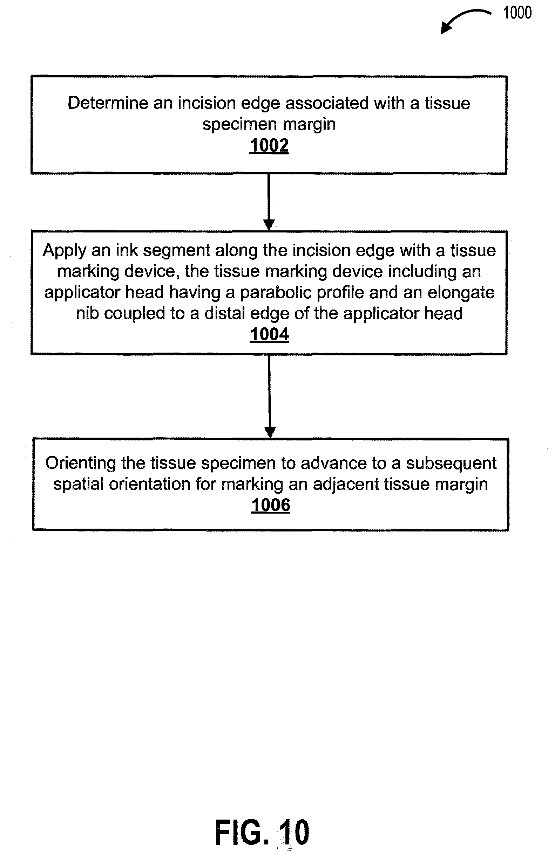

Reference is made to FIG. 10, which illustrates a flowchart 1000 of a method of tissue marking. The method of tissue marking may be conducted intraoperatively by a surgeon or a medical team member.

At operation 1002, an incision edge associated with a tissue specimen margin may be determined.

At operation 1004, an ink segment may be applied along the incision edge with a tissue marking device. In some embodiments, the tissue marking device may include an applicator head having a parabolic profile and an elongate nib coupled to a distal edge of the applicator head. The elongate nib may include a nib profile corresponding to the parabolic profile of the applicator head and may be adapted to dispense ink.

At operation 1006, the tissue specimen may be oriented to advance to a subsequent spatial orientation for marking an adjacent tissue margin.

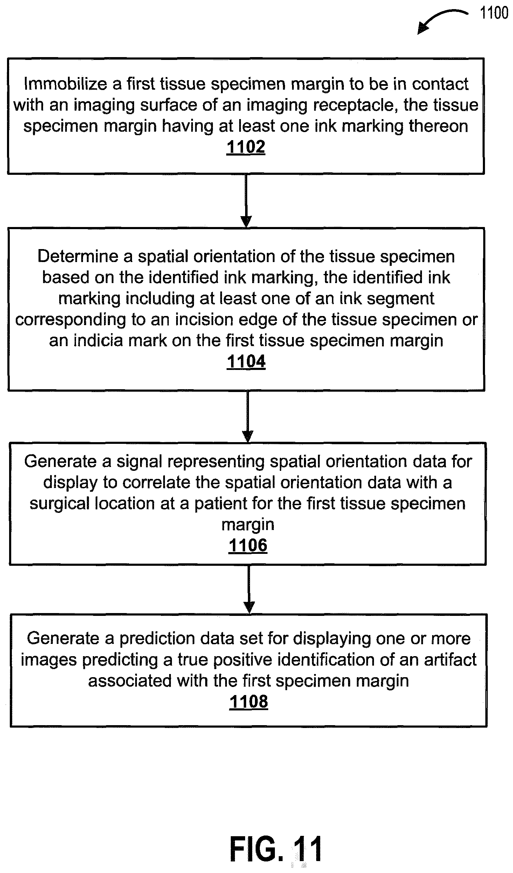

Reference is made to FIG. 11, which illustrates a flowchart of a method 1100 of tissue specimen analysis, in accordance with an embodiment of the present disclosure. The method 1100 may be conducted by one or more features of the tissue analysis system 800 described with reference to FIG. 8. Some operations may be conducted by a processor of the tissue analysis system 800.

At operation 1102, the system may immobilize a first tissue specimen margin to be in contact with an imaging surface of an imaging receptacle. The tissue specimen margin may have at least one ink marking thereon. An example imaging receptacle 810 is illustrated in FIG. 8, and may include a receptacle having an imaging surface, such as a glass surface, integrated therein.

In some embodiments, immobilizing the first tissue specimen margin includes applying a vacuum suction force adapted to draw the first tissue margin to be in contact with the imaging surface of the imaging receptacle. An example field of view of an image capture device is illustrated in FIG. 9.

In some embodiments, the tissue specimen margin may represent an excised adipose tissue specimen.

At operation 1104, the system may determine a spatial orientation of the tissue specimen based on the identified ink marking. The identified ink marking may include at least one of an ink segment corresponding to an incision edge of the tissue specimen or an indicia mark on the first tissue specimen margin.

In some embodiments, the identified ink marking may include a colored ink marking representing a coded indication of at least one of an anterior side, a posterior side, a superior side, an inferior side, a medial side, or a lateral side of the tissue specimen.

At operation 1106, the system may generate a signal representing spatial orientation data for display to correlate the spatial orientation data with a surgical location at a patient for the first tissue specimen margin.

At operation 1108, the system may generate a prediction data set for displaying one or more images predicting a true positive identification of an artifact associated with the first specimen margin.

In some embodiments, an identified artifact associated with an image may represent one or more cancerous cells associated with a tissue specimen margin. In some embodiments, the tissue specimen margin may represent an excised adipose tissue specimen, or other types of tissue.

In some embodiments, the method may include orienting the tissue specimen to advance to a subsequent spatial orientation for determining spatial orientation data for a second tissue specimen margin. In some scenarios, operations for further orienting the tissue specimen to advance to a subsequent spatial orientation may be conducted within the imaging receptacle 810 (FIG. 8). As illustrated in FIG. 9, ink segments or ink indicia may assist the system with determining approximate boundaries of a tissue specimen margin to be analyzed. Examples of tissue specimens may substantially change shape when advancing from one spatial orientation to a subsequent spatial orientation, thereby making it challenging to identify tissue specimen margins. Embodiments of marking devices for generating ink segments or ink indicia at tissue specimen margins may be desirable for supporting operations of tissue specimen analysis.

The term "connected" or "coupled to" may include both direct coupling (in which two elements that are coupled to each other contact each other) and indirect coupling (in which at least one additional element is located between the two elements).

Although the embodiments have been described in detail, it should be understood that various changes, substitutions and alterations can be made herein without departing from the scope. Moreover, the scope of the present disclosure is not intended to be limited to the particular embodiments of the process, machine, manufacture, composition of matter, means, methods and steps described in the specification.

As one of ordinary skill in the art will readily appreciate from the disclosure, processes, machines, manufacture, compositions of matter, means, methods, or steps, presently existing or later to be developed, that perform substantially the same function or achieve substantially the same result as the corresponding embodiments described herein may be utilized. Accordingly, the appended claims are intended to include within their scope such processes, machines, manufacture, compositions of matter, means, methods, or steps.

The description provides many example embodiments of the inventive subject matter. Although each embodiment represents a single combination of inventive elements, the inventive subject matter is considered to include all possible combinations of the disclosed elements. Thus if one embodiment comprises elements A, B, and C, and a second embodiment comprises elements B and D, then the inventive subject matter is also considered to include other remaining combinations of A, B, C, or D, even if not explicitly disclosed.

The embodiments of the devices, systems and methods described herein may be implemented in a combination of both hardware and software. These embodiments may be implemented on programmable computers, each computer including at least one processor, a data storage system (including volatile memory or non-volatile memory or other data storage elements or a combination thereof), and at least one communication interface.

Program code is applied to input data to perform the functions described herein and to generate output information. The output information is applied to one or more output devices. In some embodiments, the communication interface may be a network communication interface. In embodiments in which elements may be combined, the communication interface may be a software communication interface, such as those for interprocess communication. In still other embodiments, there may be a combination of communication interfaces implemented as hardware, software, and combination thereof.

Throughout the foregoing discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable tangible, non-transitory medium. For example, a server can include one or more computers operating as a web server, database server, or other type of computer server in a manner to fulfill described roles, responsibilities, or functions.

The embodiments described herein are implemented by physical computer hardware, including computing devices, servers, receivers, transmitters, processors, memory, displays, and networks. The embodiments described herein provide useful physical machines and particularly configured computer hardware arrangements.

As can be understood, the examples described above and illustrated are intended to be exemplary only.

## Claims

1. A tissue marking device comprising:
an elongate body member;
an applicator head coupled to the elongate body member having a parabolic profile; and
an elongate nib coupled to a distal edge of the applicator head, the elongate nib including a nib profile corresponding to the parabolic profile of the applicator head and adapted to dispense ink.

2. The tissue marking device of claim 1, wherein the elongate nib includes an ink-filled burstable capsule.

3. The tissue marking device of claim 1 or claim 2, wherein the applicator head includes a chamfered edge.

4. The tissue marking device of any preceding claim, wherein the elongate body member includes a plurality of grooves extending a length of the elongate body member.

5. The tissue marking device of any preceding claim, comprising a protective cover having a profile substantially similar to the applicator head and adapted to cover the applicator head.

6. The tissue marking device of any preceding claim, comprising:
an indicia head coupled to the elongate body member at a distal end to the applicator head; and
a point nib coupled to the indicia head and adapted to dispense ink.

7. The tissue marking device of claim 6, wherein the point nib includes a cylindrical profile.

8. The tissue marking device of claim 6 or claim 7, wherein the point nib includes colored ink absorbed therein.

9. A method of tissue marking comprising:
determining an incision edge associated with a tissue specimen margin;
applying an ink segment along the incision edge with a tissue marking device, the tissue marking device including an applicator head having a parabolic profile and an elongate nib coupled to a distal edge of the applicator head, wherein the elongate nib including a nib profile corresponding to the parabolic profile of the applicator head and adapted to dispense ink; and
orienting the tissue specimen to advance to a subsequent spatial orientation for marking an adjacent tissue margin.

10. The method of tissue marking of claim 9, comprising:
applying an indicia marking to the tissue specimen margin with the tissue marking device, the tissue marking device including an indicia head having a point nib adapted for applying a coded indicia to the tissue specimen margin and, optionally,
wherein the point nib includes a cylindrical profile, and wherein the indicia marking includes at least one of a color-coded marking or an alpha-numeric character.

11. The method of tissue marking of any of claims 9 to 10, wherein the elongate nib includes an ink-filled burstable capsule, and wherein the method comprises: breaching the ink-filled burstable capsule.

12. A method of tissue specimen analysis comprising:
immobilizing a first tissue specimen margin to be in contact with an imaging surface of an imaging receptacle, the tissue specimen margin having at least one ink marking thereon;
determining a spatial orientation of the tissue specimen based on the identified ink marking, the identified ink marking including at least one of an ink segment corresponding to an incision edge of the tissue specimen or an indicia mark on the first tissue specimen margin;
generate a signal representing spatial orientation data for display to correlate the spatial orientation data with a surgical location at a patient for the first tissue specimen margin.

13. The method of tissue specimen analysis of claim 12, comprising:
generating a prediction data set for displaying one or more images predicting a true positive identification of an artifact associated with the first specimen margin.

14. The method of claim 13, wherein the identification of the artifact associated with the first specimen margin represents identification of cancerous cells at or proximal to the tissue specimen margin.

15. The method of tissue specimen analysis of claim 12, wherein the identified ink marking includes a colored ink marking representing a coded indication of at least one of an anterior side, a posterior side, a superior side, an inferior side, a medial side, or a lateral side of the tissue specimen.

16. The method of tissue specimen analysis of claim 12, wherein the tissue specimen margin represents an excised adipose tissue specimen.

17. The method of tissue specimen analysis of claim 12, wherein immobilizing the first tissue specimen margin includes applying a vacuum suction force adapted to draw the first tissue margin to be in contact with the imaging surface of the imaging receptacle.

18. The method of tissue specimen analysis of claim 12, comprising: orienting the tissue specimen to advance to a subsequent spatial orientation for determining spatial orientation data for a second tissue specimen margin.
